Europäisches Patentamt

**European Patent Office**

**Office européen des brevets**

(11) Publication number: **0 057 659**
A2

# EUROPEAN PATENT APPLICATION

(21) Application number: **82730007.0**

(22) Date of filing: **29.01.82**

(51) Int. Cl.³: **B 01 J 10/00, C 12 M 1/04**

(30) Priority: **30.01.81 DE 3103681**

(43) Date of publication of application: **11.08.82**
**Bulletin 82/32**

(84) Designated Contracting States: **DE FR GB IT NL**

(71) Applicant: **SCHERING AKTIENGESELLSCHAFT Berlin und Bergkamen,**
**Müllerstrasse 170/178 Postfach 65 03 11,**
**D-1000 Berlin 65 (DE)**

(72) Inventor: **Müller, Rudolf, Dr., Corneliusstrasse 17,**
**D-1000 Berlin 46 (DE)**

(54) **Draft tube type reactor with centrally arranged auxiliary agitator.**

(57) A draft tube reactor adapted for conducting fermentation processes includes a draft tube arranged within the reactor housing and extending almost along the entire lenght of the reactor housing. An injector is arranged immediately below the draft tube for injecting oxygen containing gas bubbles thereinto so that when the reactor is filled with a liquid to a level above the top edge of the draft tube, a circulating flow of the gas bubbles and liquid is created. An agitator is located at about the top edge of the draft tube for breaking up large bubbles formed as a result of coalescence of the bubbles injected into the draft tube to thereby enhance oxygen circulation and exchange within the fluid. The invention also comprises a method of enhancing oxygen circulation and exchange in a liquid in a reactor.

0057659

## DRAFT TUBE TYPE REACTOR WITH
## CENTRALLY ARRANGED AUXILIARY AGITATOR

### BACKGROUND OF THE INVENTION

This invention relates to a draft tube mixer type reactor, and more specifically, the invention relates to a fermentor for use in conducting fermentation processes.

The prior art teaches the use of specially designed fermentors for industrial use in conducting fermentations. These fermentors are constructed so that they continuously agitate the material to be fermented. To accomplish the continuous agitation, it is known to provide an agitating system having a continuous shaft with stirring means extending throughout the length of the fermenting chamber. Typically, such a shaft can be mounted on the bottom of the fermentor. However, for large-scale fermentation processes, it is preferable to use a draft tube mixer type reactor, i.e., a bubble column reactor or loop column reactor, because they provide advantages over the prior art fermentors having mechanical agitating means when employed in industrial applications.

In fermentation processes, it is essential that oxygen fed into the fermentor be maximally distributed for use in the progression of the chemical reaction. In order to achieve satisfactory oxygen exchange, fermentors are constructed to be relatively long and/or tall in order to maintain a long residence time of the oxygen containing gas bubbles in the liquid being fermented.  In the fermentors having mechanical agitating means, the size of the reactor is limited to a volume of 200-300 m$^3$ because of limitations on the size of the shaft possible for use.  However, in the case of draft tube mixer type reactors, there are no limits with respect to volume.  Furthermore, draft tube type reactors are also of a simple construction due to the elimination of the mechanical agitating means extending therethrough and result in a savings in electricity and cooling water.  Moreover, the maintenance expense is also substantially lower than in reactors having agitating means due to a reduced number of movable parts.

The intermixing of the fermentation liquid is effected in draft tube type mixer by a rising stream of bubbles conducted through a draft tube arranged in the mixer so as to direct a stream of bubbles injected into the mixer upwardly therethrough.  However, it has been found that as the air bubbles rise in the liquid, the small air bubbles will tend to coalesce and combine to form relatively large air bubbles.  Because large gas bubbles provide a relatively smaller surface area for the equivalent volume distributed throughout a plurality of smaller gas bubbles, there is a reduced gas exchange than that achieved with small gas bubbles.  Furthermore, the large gas bubbles containing substantial amounts of oxygen flow upward through the liquid and are released from the surface of the liquid.  Thus, the exhausted air still contains relatively high concentrations of oxygen and the process results in an inefficient use of the oxygen in the air fed thereinto.

In contrast, the smaller bubbles, which at the surface of the liquid in the reactor contain relatively low amounts of oxygen due to efficient utilization of

the $O_2$ content as they flow upwardly through the liquid, are preferentially recycled by the refluxing liquid to the bottom of the fermentor. Thus, the supply of oxygen supply in the reflux portion of the process is low and can only be compensated for by injection of excess amounts of air during gasification, i.e., gas injection at the bottom of the reactor. However, by injecting large amounts of air the probability of large gas bubbles forming is also increased which in turn further reduces the efficiency of the process.

## SUMMARY OF THE INVENTION

It is thus an object of the present invention to provide a draft tube type reactor having improved oxygen utilization.

It is another object of the invention to provide a draft tube type reactor having improved oxygen utilization by arranging agitating means operatively associated with the draft tube thereof.

Another object of the invention is to provide a draft tube type reactor adapted for use in fermentation processes and having means for improving oxygen utilization therein.

Still another object of the invention is to provide a method of increasing oxygen utilization in a draft tube type reactor.

Upon further study of the specification and appended claims, further objects and advantages of this invention will become apparent to those skilled in the art.

These and other objects according to the invention are achieved providing an agitator centrally within the reactor in the upper zone of the draft tube. In the reactor, the draft tube serves to cause an agitating flow of the gas bubbles within the liquid to enhance oxygen exchange in the fermentor and cause circulation of the liquid therein. However, as the bubbles rise through the draft tube they tend to coalesce and form large bubbles which escape from the liquid and are not recirculated. Thus, in accordance with the invention there is provided an agitating means associated with the draft tube in the upper region, e.g., about the top edge, thereof for breaking up the large bubbles.

### BRIEF DESCRIPTION OF THE DRAWINGS

Various other objects, features and attendant advantages of the present invention will be more fully appreciated as the same becomes better understood when considered in conjunction with the accompanying drawings, in which like reference characters designate the same or similar parts throughout the several views, and wherein:

FIGURE 1 illustrates the agitator located immediately above the top edge, i.e., between the surface of the liquid and the top edge of the draft tube.

FIGURE 2 schematically illustrates one embodiment of the invention employing the agitator located within the interior of the reactor within the draft tube and immediately below the top edge thereof.

### DETAILED DISCUSSION OF THE INVENTION

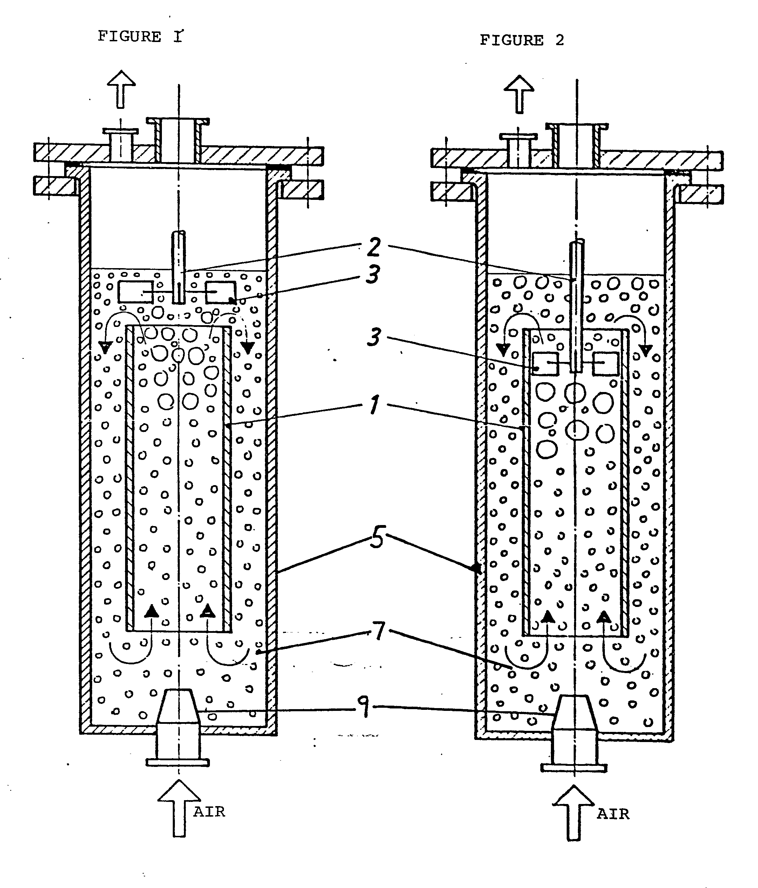

Figures 1 and 2 illustrate a draft tube type reactor 5 incorporating the features of the invention. More specifically, the reactor 5 is shown filled with liquid 7 through which oxygen containing gas bubbles, i.e., preferably air, are injected by injecting means 9. The injecting means 9 and draft tube 1 are positioned centrally within the reactor 5. Furthermore, the injecting means 9 is positioned directly beneath the draft tube 1 to cause gas bubbles to flow upwardly therethrough and to cause a circulating flow of fluid and bubbles in the reactor 5 as shown by the arrows in Figures 1 and 2.

An agitator 3 is positioned at any desired location between the two positions shown in Figures 1 and 2. More specifically, the same effect is achieved if the agitator 3 is used at approximately the level of the top edge of the draft tube 1 as is achieved with the agitator 3 located at a level immediately below or above the top edge of the draft tube 1. Generally, the agitator 3 is located at a distance above the draft tube top no more than 0.01 to 0.13 the length of the draft tube, preferably 0.1 the length of the tube, and at a distance below the draft tube top no more than 0.01 to 0.13 the length of the tube, preferably 0.1, measured at the central fixing point on the agitator shaft 2.

Thus, by maintaining the elements of the device within these ranges, it is ensured that, (1) the large air bubbles broken up by the agitator 3 do not coalesce again into large bubbles before being recirculated downward, and (2) are broken up sufficiently below the surface of the liquid to ensure that virtually no large bubbles escape breaking up by the agitator 3. Likewise, by maintaining the draft tube positioned within the above ranges, it is ensured that a maximum circulation of the bubbles and fluid are achieved. In this regard, the remaining details about the dimensions of the reactor are conventional in nature and can be readily determined as discussed in, e.g., Industrielle Mikrobiologie, Springer-Verlag, Heidelberg-Berlin-New York, 1980, incorporated by reference herein.

In this regard, for the draft tube reactor of the invention to funktion properly, the draft tube top must be located at a level between 1/5 to 1/20, preferably 1/10 to the total hight of the reactor vessel below the surface of the liquid in the reactor.

The agitator 3 serves to break up the large air bubbles so that the thus-produced, smaller bubbles are entrained to a larger degree by the reflux in the direction toward the fermentor bottom. Furthermore, in the invention the agitator shaft 2 of the agitator 3 is relatively short, so that a vibration-free operation is possible even with a high number of revolutions.

The bubble size and the oxygen content of the liquid depends on many factors, e.g., the specific liquid, temperature, air flow, number of revolutions, etc.

0057659

In accordance with the invention the fermentors exhibit a substantially improved oxygen exchange as compared to the prior art. Thus, the feed air for the gasification can therefore be relatively reduced in quantity. The energy consumption, when compared to a conventional stirred fermentor, is substantially reduced. Furthermore, the agitator 3 employed can comprise any one of various conventional agitator elements, such as a turbine, disk, MIG (multistage impulse countercurrent agitator), crossbar stirrers, liquid-intake stirrers, blade stirrers, or other conventional agitating systems.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. A draft tube reactor comprising, housing means for containing a liquid to be treated therein, draft tube means arranged centrally within said housing means spaced upwardly from the bottom thereof, gas injecting means arranged so as to inject gas bubbles through the liquid in said housing means and into said draft tube means, and agitating means arranged near the top of said draft tube means for breaking up large bubbles resulting from coalescence of the gas bubbles injected by said injecting means and to enhance circulation of the gas bubbles through the fluid in said housing means.

2. A draft tube reactor according to claim 1, wherein said agitating means is mounted below and near the top edge of said draft tube means.

3. A draft tube reactor according to claim 1, wherein said agitating means is mounted above and near the top edge of said draft tube means.

4. A draft tube reactor according to claim 1, wherein said agitating means is mounted no higher than 0.13 the length of the draft tube above the top edge than said draft tube means, and no lower than 0.13 the length of the draft tube below the top edge of said draft tube means.

5.   A draft tube reactor according to claim 1, wherein said agitating means comprises at least one of a turbine, disk, MIG, crossbar stirrer, liquid intake stirrer, and blade stirrer.

6.   A draft tube reactor according to claim 1, wherein said reactor is a fermentor.

7.   In a method of conducting a fermentation process in a draft tube reactor wherein oxygen containing gas is injected into a draft tube extending lengthwise within the reactor for causing a circulating flow of a fluid and the gas bubbles therein, the improvement comprising breaking up large bubbles, formed as a result of coalescence of the bubbles injected into the reactor, at a position about the top of the draft tube.

FIGURE 1

FIGURE 2

AIR

AIR